# EUROPÄISCHE PATENTSCHRIFT

(11) **EP 0 656 199 B1**
(45) Veröffentlichungstag und Bekanntmachung des Hinweises auf die Patenterteilung: **12.12.2001**
(21) Anmeldenummer: 94116607.6
(22) Anmeldetag: 21.10.1994
(51) Int. Cl.: A61F 2/34, A61L 27/00

(54) **Sphärische Hüftgelenkpfanne**
Spherical hip joint acetabular cup
Coque acétabulaire sphérique pour l'articulation de la hanche

(30) Priorität: 06.11.1993 DE 4337936
(43) Veröffentlichungstag der Anmeldung: 07.06.1995
(73) Patentinhaber: CERASIV GmbH INNOVATIVES KERAMIK-ENGINEERING, D-73207 Plochingen (DE)
(72) Erfinder: Kälberer, Hartmut, Dipl.-Ing. (BA), D-73779 Deizisau (DE); Pfaff, Hans-Georg, Dipl.-Ing. (FH), D-73160 Ostfildern (DE)
(74) Vertreter: Scherzberg, Andreas, Dr.

(56) Entgegenhaltungen:
- EP-A- 0 123 514
- EP-A- 0 208 578
- EP-A- 0 253 941
- EP-A- 0 444 382
- EP-A- 0 445 068
- WO-A-86/02261
- DE-A- 3 215 583
- DE-A- 3 838 568
- FR-A- 2 242 065
- FR-A- 2 645 433
- FR-A- 2 682 588
- FR-A- 2 700 686
- GB-A- 2 126 096

## Beschreibung

Die Erfindung betrifft eine Hüftgelenkpfanne nach dem Oberbegriff des Anspruchs 1. Eine Pfanne nach dem Oberbegriff von Anspruch 1 ist beispielsweise aus der FR-A- 2 682 588 bekannt.

Hüftgelenk-Endoprothesen bestehen aus einer Hüftgelenkpfanne, die im Beckenknochen verankert ist und aus einer Kugel, die in die Pfanne drehbar eingesetzt ist und mit einem Schaft im Oberschenkelknochen verankert ist.

Hüftgelenkpfannen bestehen aus einer äußeren Metallschale, welche die Implantataußenkontur darstellt und aus einer inneren Schale, die aus Keramik hergestellt ist. Die Keramikschale ist in der Metallschale mechanisch verankert. Es gibt auch innere Schalen aus Kunststoff. Der Begriff Metallschale steht synonym für den metallischen Teil des Acetabular Implantates, dessen Außenkontur den medizinischen Anforderungen entsprechend gestaltet werden kann.

Es ist Stand der Technik, die innere Keramikschale in der Metallschale mit Hilfe einer konischen Klemmung zu fixieren. Der Winkel der konischen Klemmung liegt dabei bei 5° 43', d.h. einem Winkelverhältnis von 1:10.

Nachteil dieser konischen Klemmung ist, daß eine einmal inserierte Keramikschale nicht mehr aus der Metallschale entfernt werden kann, weil die Klemmkräfte zu hoch sind. Damit kann ein Austausch der Gleitlagerkomponente, d.h. der Keramikschale während der Operation oder im Falle einer Reoperation nicht mehr ohne weiteres erfolgen. Üblicherweise muß dann das gesamte Pfannenimplantat entfernt oder das keramische Inlay zerstört werden. Aufgrund der Splitterbildung führt dies jedoch zu Problemen.

Ein weiterer Nachteil der konischen Klemmung ist eine ungünstige Belastungssituation für die Keramikkomponente. Diese wird an dem äußeren konischen Ring eingespannt und ist dann an der Pfannengewölbeseite freitragend, was zu erheblichen Zugbeanspruchungen der Komponente führt. Um ein Pfanneninlay den mechanischen Belastungen entsprechend auszulegen, sind erhebliche Wandstärken (mindestens 4 - 5 mm) erforderlich. Dies hat zur Folge, daß die Keramik-Metall-Pfannen große Außendurchmesser aufweisen. Von medizinischer Seite jedoch besteht die Forderung nach kleinen Komponenten, um den Knochenverlust möglichst minimal zu halten.

Der Erfindung liegt die Aufgabe zugrunde, eine Hüftgelenkpfanne nach dem Oberbegriff des Anspruchs 1 derart weiterzubilden, daß sie bei einer dünnen Wandstärke der Keramikschale eine geringe Baugröße aufweist.

Erfindungsgemäß wird die Aufgabe mit einer Hüftgelenkpfanne nach Auspruch 1 gelöst.

Durch die paßgenaue sphärische Innenkontur der Metallschale und der Außenkontur der Keramikschale kann die Keramikschale extrem dünnwandig ausgebildet werden, da alle Krafteinwirkungen auf die Metallschale verteilt werden. Hierdurch ist die Baugröße des Implantats äußerst klein, so daß wenig Knochenmasse vom Operateur entfernt zu werden braucht.

Um Spannungsspitzen, die aufgrund von technisch bedingten und nicht vermeidbaren Maßabweichungen auftreten, kompensieren zu können, werden erfindungsgemäß Mittel zur Homogenisierung der Kraftübertragung zwischen der Keramikschale und der Metallschale angeordnet.

Gemäß der Erfindung ist das Mittel zur Homogenisierung der Kraftübertragung eine auf der Innenseite der Metallschale aufgebrachte Oberflächenaufrauhung, wodurch die Oberfläche plastisch verformbar ist. Durch diese Maßnahme kann sich die Oberfläche der Metallschale verformen und Maßungenauigkeiten kompensieren.

Erfindungsgemäß weist die Oberflächenaufrauhung eine Rauhigkeit von 4 µm bis 100 µm auf, ausgenommen 4 µm. Bevorzugt ist eine Rauhigkeit von 20 µm bis 40 µm.

Vorteilhafterweise kann die Oberflächenaufrauhung eine z.B. durch Schleifen erzeugte unregelmäßige Struktur aufweisen.

Bevorzugt ist eine regelmäßige Struktur der Oberflächenaufrauhung, die z.B. durch Drehen erzeugt wird.

In bevorzugter Ausführungsform besteht die regelmäßige Struktur aus konzentrischen Kreisen oder weist eine durch Drehen hergestellte spiralige Form auf.

Die Keramikschale ist in der Metallschale über einen Haltering verankert, der auf dem Rand der Metallschale befestigt ist.

Weitere Merkmale der Erfindung zeigen die Figuren, die nachfolgend beschrieben sind. Es zeigt:
- Fig. 1: in zwei Schnitten eine erfindungsgemäße Metallschale mit einer aufgebrachten Oberflächenaufrauhung,
- Fig. 2: einen Ausschnitt X aus Fig. 1a mit einer Sägezahnstruktur,
- Fig. 3: eine erfindungsgemäße Metallschale mit angebrachtem Haltering,
- Fig. 4: einen Ausschnitt X aus Fig. 3 vergrößert dargestellt,
- Fig. 5: einen Haltering zur Festlegung der Keramikschale in der Metallschale,
- Fig. 6: eine erfindungsgemäße Keramikschale im Schnitt,
- Fig. 7: schematisch auf der Innenseite der Metallschale aufgebrachte konzentrische Ringe, und
- Fig. 8: schematisch eine auf der Innenseite der Metallschale aufgebrachte spiralige Oberflächenaufrauhung.

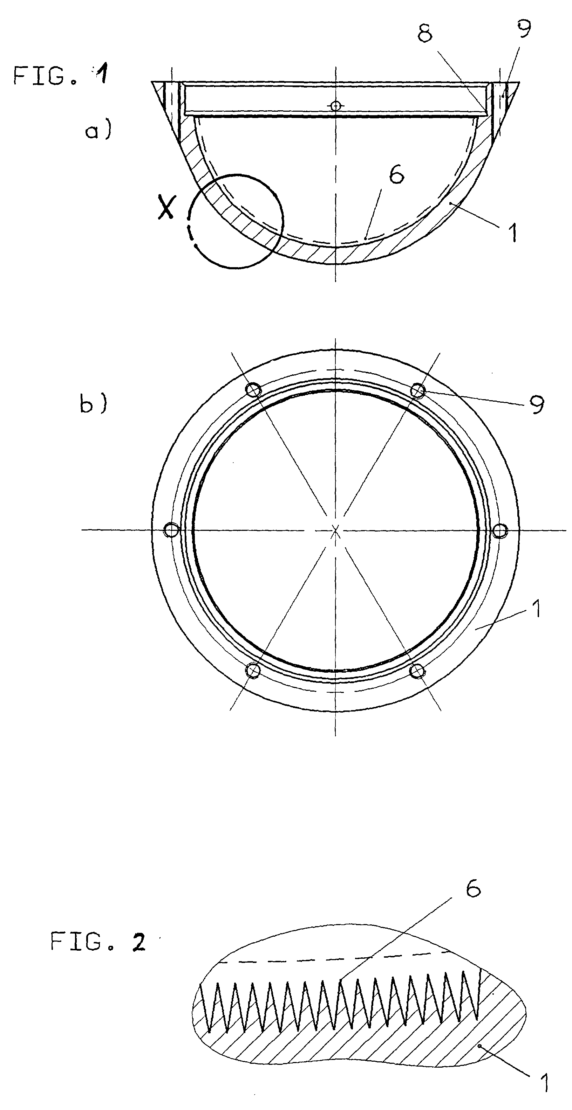

Fig. 1a, 1b zeigt eine Ausführungsform, bei der zur Homogenisierung der Kraftübertragung auf der Innenseite der Metallschale 1 eine Oberflächenaufrauhung 6 aufgebracht ist, wodurch die Oberfläche plastisch verformbar ist. Fig. 1a zeigt im Schnitt eine erfindungsgemäße Metallschale 1, deren Stirnseite eine Ausbuchtung 8 für einen Kragen einer Keramikschale aufweist. Auf dem Rand der Metallschale 1 sind in Längsrichtung Bohrungen 9 angeordnet, die zur Befestigung eines Halteringes dienen. Fig. 1b zeigt eine Draufsicht auf die Stirnseite der Metallschale 1 gemäß Fig. 1a.

Die Oberflächenaufrauhung 6 weist eine Rauhigkeit von 4 µm bis 100 µm auf. Besonders vorteilhaft ist eine Rauhigkeit von 20 µm bis 40 µm.

Beim Einlegen der Keramikschale (Beschreibung siehe später) verformt sich die Oberflächenaufrauhung 6 plastisch, so daß hierdurch Spannungspitzen aufgefangen werden.

Fig. 2 zeigt vergrößert eine Oberflächenaufrauhung 6 in der Form eines Sägezahnprofils. Die Oberflächenaufrauhung 6 kann z.B. durch Schleifen erzeugt werden, was eine unregelmäßige Struktur bewirkt. Die Oberflächenaufrauhung 6 kann vorteilhafterweise auch durch z.B. Drehen aufgebracht werden, wodurch eine regelmäßige Struktur erzeugt wird.

Fig. 7 zeigt als bevorzugte Ausführungsform konzentrische Kreise 4 als Oberflächenaufrauhung. Es sind jedoch auch andere Strukturen möglich, wie z.B. eine Spirale. Eine solche Oberflächenaufrauhung zeigt Fig. 8. Die spiralförmige Oberflächenaufrauhung ist mit dem Bezugszeichen 12 gekennzeichnet.

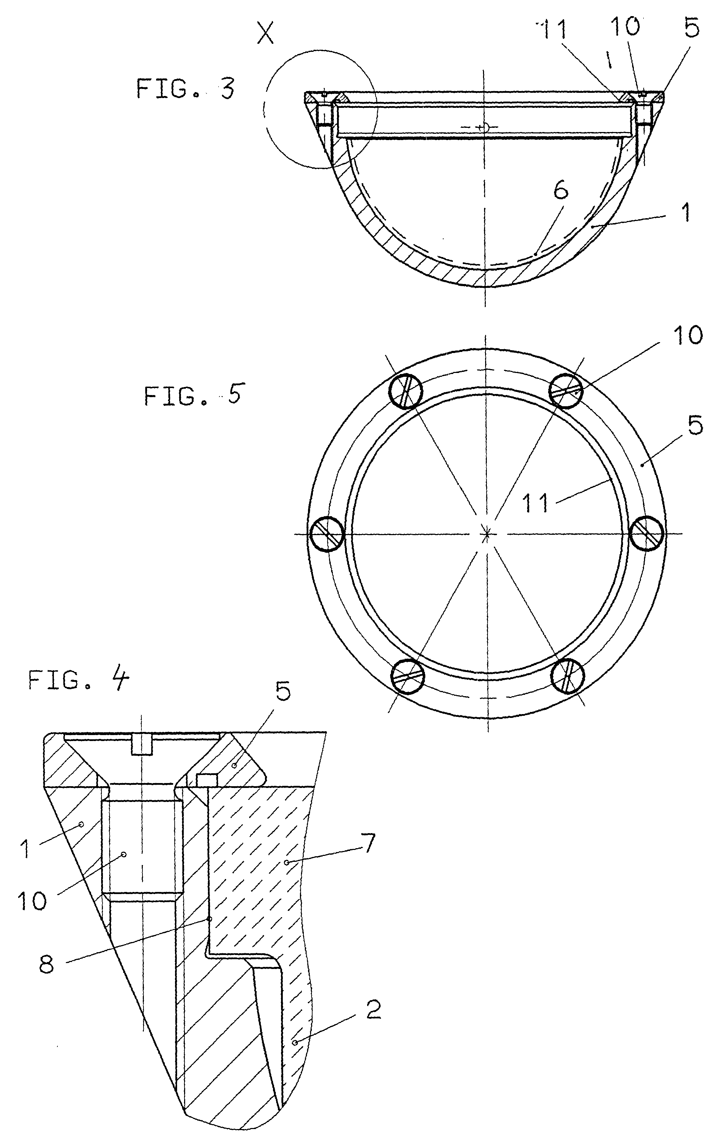

In Fig. 3 ist die erfindungsgemäße Metallschale 1 mit aufgesetztem Haltering 5 gezeigt. Der Haltering 5 ist mit Schrauben 10 auf der Metallschale 1 befestigt. Die Schrauben 10 sind dabei in die schon in Fig. 1a beschriebenen Bohrungen 9 eingeschraubt.

In Fig. 5 ist der Haltering 5 dargestellt mit eingesetzten Schrauben 10. Der Vorsprung 11 sitzt auf dem hier nicht gezeigten Keramikring auf und preßt ihn in die Metallschale 1.

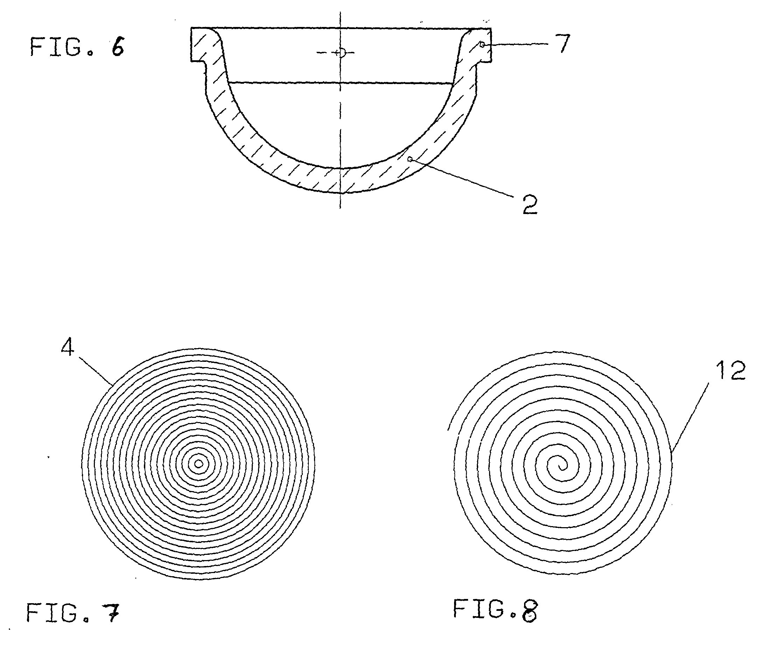

Fig. 4 zeigt den Ausschnitt X aus Fig. 3 vergrößert dargestellt mit eingesetztem Keramikring 2. Die Keramikschale 2 sitzt mit ihrer Auskragung 7 in der Ausbuchtung 8 und wird vom Haltering 5 in die Keramikschale 2 gepreßt. Während des Einpreßvorgangs verformt sich die Oberflächenaufrauhung 6 der Metallschale 1 plastisch.

Fig. 6 zeigt den Keramikring 2 mit seiner Auskragung 7.

Die erfindungsgemäße Hüftgelenkpfanne zeichnet sich durch eine extrem kleine Baugröße aus. Ein weiterer Vorteil ist, daß die Keramikschale 2 einfach durch Abschrauben des Halteringes 5 auch während einer Operation ausgewechselt werden kann.

## Patentansprüche

1. Hüftgelenkpfanne zum Einsetzen in Knochengewebe mit einer äußeren Metallschale (1) und einer inneren Keramikschale (2), wobei die Keramikschale (2) in der Metallschale (1) verankert ist, und Mittel zur Homogenisierung der Kraftübertragung zwischen der Keramikschale (2) und der Metallschale (1) angeordnet sind, **dadurch gekennzeichnet, daß** die Innenseite der Metallschale (1) und die Außenseite der Keramikschale (2) eine aneinander angepaßte paßgenaue sphärische Kontur aufweisen, und daß die Mittel zur Homogenisierung der Kraftübertragung eine auf der Innenseite der Metallschale (1) aufgebrachte Oberflächenaufrauhung (6) ist, wodurch die Oberfläche plastisch verformbar ist und die Oberflächenaufrauhung (6) eine Rauhigkeit von 4 µm bis 100 µm aufweist, ausgenommen eine Rauhigkeit von 4 µm, und daß ein auf dem Rand der Metallschale (1) befestigter Haltering (5) die Keramikschale (2) in der Metallschale (1) verankert.

2. Hüftgelenkpfanne nach Anspruch 1, **dadurch gekennzeichnet, daß** die Oberflächenaufrauhung (6) eine Rauhigkeit von 20 µm bis 40 µm aufweist.

3. Hüftgelenkpfanne nach Anspruch 1 oder 2, **dadurch gekennzeichnet, daß** die Oberflächenaufrauhung (6) eine z. B. durch Schleifen erzeugte unregelmäßige Struktur aufweist.

4. Hüftgelenkpfanne nach Anspruch 1 oder 2, **dadurch gekennzeichnet, daß** die Oberflächenaufrauhung (6) eine z. B. durch Drehen erzeugte regelmäßige Struktur aufweist.

5. Hüftgelenkpfanne nach Anspruch 4, **dadurch gekennzeichnet, daß** die regelmäßige Struktur konzentrische Kreise (4) sind.

6. Hüftgelenkpfanne nach Anspruch 4, **dadurch gekennzeichnet, daß** die regelmäßige Struktur eine durch Drehen hergestellte spiralige Form (12) aufweist.

## Claims

1. Hip-joint socket for insertion into bone tissue having an outer metal shell (1) and an inner ceramic shell (2), with the ceramic shell (2) being anchored in the metal shell (1) and with means for homogenizing the force-transmission being arranged between the ceramic shell (2) and the metal shell (1), **characterized in that** the inside of the metal shell (1) and the outside of the ceramic shell (2) have spherical contours that are adapted to each other and are an exact fit, and **in that** the means for homogenizing the force transmission is a surface roughening (6) which is applied to the inside of the metal shell (1), whereby the surface is plastically deformable and the surface roughening (6) has a roughness of 4 µm to 100 µm, excepting a roughness of 4 µm, and **in that** a holding ring (5), which is secured to the edge of the metal shell (1), anchors the ceramic shell (2) in the metal shell (1).

2. Hip-joint socket according to claim 1, **characterized in that** the surface roughening (6) has a roughness of 20 µm to 40 µm.

3. Hip-joint socket according to claim 1 or 2, **characterized in that** the surface roughening (6) has an irregular structure which is produced, for example, by grinding.

4. Hip-joint socket according to claim 1 or 2, **characterized in that** the surface roughening (6) has a regular structure which is produced, for example, by turning.

5. Hip-joint socket according to claim 4, **characterized in that** the regular structure is constituted by concentric circles (4).

6. Hip-joint socket according to claim 4, **characterized in that** the regular structure has a spiral shape (12) that is produced by turning.

## Revendications

1. Coque acétabulaire sphérique destiné à être implantée dans un tissu osseux comprenant une coque métallique (1) extérieure et une coque céramique (2) intérieure, la coque céramique (2) étant ancrée dans la coque métallique (1), ainsi que des moyens pour homogénéiser la transmission des efforts entre la coque en céramique (2) et la coque métallique (1), **caractérisée en ce que** la face intérieure de la coque métallique (1) et la face extérieure de la coque céramique (2) présentent une forme sphérique très précise mutuellement adaptée, **en ce que** le moyen pour homogénéiser la transmission des efforts est un grenage superficiel (6) appliqué sur la face intérieure de la coque métallique (1), grâce auquel la surface peut se déformer plastiquement, et **en ce que** le grenage superficiel (6) présente une rugosité comprise entre 4 µm et 100 µm, à l'exception d'une rugosité de 4 µm, et **en ce qu'**une bague de retenue (5) fixée sur le bord de la coque métallique (1) ancre la coque céramique (2) dans la coque métallique (1).

2. Coque acétabulaire sphérique selon la revendication 1, **caractérisée en ce que** le grenage superficiel (6) présente une rugosité comprise entre 20 µm et 40 µm.

3. Coque acétabulaire sphérique selon la revendication 1 ou 2, **caractérisée en ce que** le grenage superficiel (6) présente une structure irrégulière obtenue par exemple par meulage.

4. Coque acétabulaire sphérique selon la revendication 1 ou 2, **caractérisée en ce que** le grenage superficiel (6) présente une structure régulière obtenue par exemple par tournage.

5. Coque acétabulaire sphérique selon la revendication 4, **caractérisée en ce que** la structure régulière est formée de cercles (4) concentriques.

6. Coque acétabulaire sphérique selon la revendication 4, **caractérisée en ce que** la structure régulière présente une forme de spirale (12), - obtenue par tournage.
